# FASCICULE DE BREVET EUROPEEN

(11) **EP 0 870 491 B1**
(45) Date de publication et mention de la délivrance du brevet: **28.02.2001**
(21) Numéro de dépôt: 98400717.9
(22) Date de dépôt: 26.03.1998
(51) Int. Cl.: A61K 7/06, A61K 7/48, A61K 7/50

(54) **Compositions cosmétiques détergentes et utilisation**
Kosmetische Tensid Zusammensetzung sowie ihre Verwendung
Cosmetic detergent compositions and their use

(30) Priorité: 07.04.1997 FR 9704219
(43) Date de publication de la demande: 14.10.1998
(73) Titulaire: L'OREAL, 75008 Paris (FR)
(72) Inventeur: Decoster, Sandrine, 93800 Epinay sur Seine (FR); Beauquey, Bernard, 92110 Clichy (FR)
(74) Mandataire: Le Blainvaux Bellegarde, Françoise

(56) Documents cités:
- EP-A- 0 521 748
- WO-A-93/08787
- WO-A-94/06403
- WO-A-95/01152
- WO-A-96/32919

## Description

La présente invention concerne de nouvelles compositions cosmétiques à propriétés améliorées destinées simultanément au nettoyage, au conditionnement des matières kératiniques et comprenant, dans un support cosmétiquement acceptable, une base lavante constituée de tensioactifs à pouvoir détergent dans laquelle sont également présents à titre d'agents conditionneurs, des polymères cationiques en association avec des silicones aminées. L'invention concerne aussi l'utilisation desdites compositions dans l'application cosmétique susmentionnée.

Pour le nettoyage et/ou le lavage de la peau ou des cheveux, l'utilisation de compositions détergentes à base essentiellement d'agents tensioactifs classiques de type notamment anioniques, non-ioniques et/ou amphothères, mais plus particulièrement de type anioniques, est courante. Ces compositions sont appliquées sur la peau ou les cheveux mouillés et la mousse générée par massage ou friction avec les mains permet, après rinçage à l'eau, l'élimination des diverses salissures initialement présentes sur la peau ou les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage, une douceur et un lissage nettement accrus par rapport à ce qui peut être obtenu avec les compositions nettoyantes correspondantes qui en sont exemptes.

Ainsi, le brevet WO 95/01152 décrit-il une composition détergente contenant un polymère cationique, une silicone et un mélange de tensio-actifs, dont un anionique.

Sur des cheveux sensibilisés, afin d'obtenir les effets cosmétiques des silicones sur toute la longueur de la fibre capillaire, on utilise de préférence des associations de silicones et de polymères cationiques.

Toutefois, et malgré les progrès réalisés récemment dans le domaine des shampooings à base de polymères cationiques et de silicones, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-dessus, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en utilisant (A) une base lavante spécifique et (B) un système conditionneur comprenant au moins un polymère cationique particulier et au moins une silicone aminée, tels que définis ci-après, il est possible d'obtenir des compositions détergentes présentant d'excellentes propriétés cosmétiques, en particulier le démêlage, la douceur, la souplesse et le volume des cheveux traités et ceci tout en conservant leur bon pouvoir lavant intrinsèque.

Ainsi, selon la présente invention, il est maintenant proposé de nouvelles compositions détergentes comprenant, dans un milieu cosmétiquement acceptable, (A) une base lavante comprenant au moins un tensioactif anionique choisi parmi les sels des alkyléther sulfates, alkylamidoéther sulfates, alkylaryléther sulfates; alkyléther sulfosuccinates, le radical alkyle ou acyle de tous ces différents composés comportant de préférence de 8 à 24 atomes de carbone, et le radical aryle désignant de préférence un groupement phényle ou benzyle, et au moins un tensioactif amphotère de type alkylbétaïne en C₈-C₂₀ et (B) un système conditionneur comprenant au moins une silicone aminée et au moins un polymère cationique choisis parmi les homopolymères comportant comme constituant principal de la chaîne des motifs répondant aux formules (I) et/ou (I'): 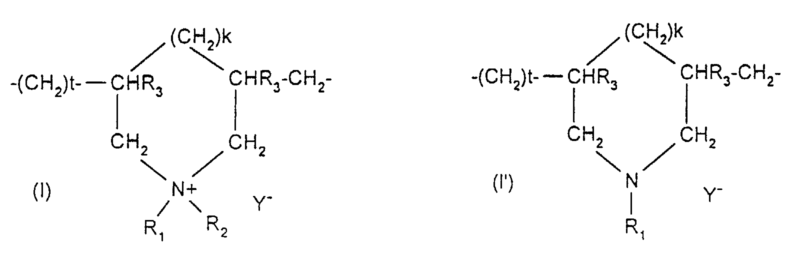 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

L'invention a également pour objet l'utilisation en cosmétique des compositions ci-dessus pour le nettoyage, le conditionnement des matières kératiniques telles que les cheveux et la peau.

Les compositions peuvent par exemple être utilisées pour le démaquillage des matières kératiniques telles que la peau (par exemple du visage, du cou ou des lèvres), les cils, les sourcils.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (A) une base lavante, (B) un système conditionneur comprenant (i) au moins une silicone aminée et (ii) au moins un polymère cationique particulier.

### A- BASE LAVANTE :

Les compositions conformes à l'invention comprennent nécessairement une base lavante, généralement aqueuse, comprenant un ou plusieurs tensioactifs anioniques choisi parmi les sels des alkyléther sulfates, alkylamidoéther sulfates, alkylarylether sulfates; alkyléther sulfosuccinates, le radical alkyle ou acyle de tous ces différents composés comportant de préférence de 8 à 24 atomes de carbone, et le radical aryle désignant de préférence un groupement phényle ou benzyle, et un ou plusieurs tensioactifs amphotères alkylbétaïne en C₈-C₂₀.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 2 % à 50 % en poids, de préférence de 10 % à 35 % en poids, et encore plus préférentiellement de 12 % à 25 % en poids, du poids total de la composition finale.

### (i) Tensioactif(s) anionique(s) de type alkyl éthersulfate :

Les tensioactifs anioniques utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkyléthersulfates, alkylamidoéthersulfates, alkylaryléthersulfates; les alkyléthersulfosuccinates, le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène pouvant aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent notamment de 2 à 5 groupements d'oxyde d'éthylène.

Les tensioactifs anioniques sont généralement présents à raison de 1 à 50 % en poids, de préférence de 5 à 20 % en poids, par rapport au poids total de la composition.

### (ii) Tensioactif(s) amphotère(s) :

Selon l'invention, les agents tensioactifs amphotères doivent être choisis parmi les alkyl (C₈-C₂₀) bétaïnes de formule : 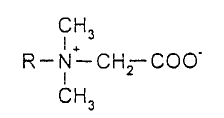 dans laquelle R désigne un radical alkyle, linéaire ou ramifié, en C₈-C₂₀, de préférence en C₁₀-C₁₄, et plus particulièrement en C₁₂-C₁₄.

En particulier, on préfère utiliser la cocobétaïne commercialisée par la société HENKEL sous la dénomination DEHYTON® AB 30.

Les tensioactifs amphotères sont généralement présents à raison de 1 à 50 % en poids, de préférence de 1 à 20 % en poids et plus particulièrement de 2 à10 % en poids par rapport au poids total de la composition.

### B) SYSTEME CONDITIONNEUR

### (i)- Silicones aminées

Selon une caractéristique essentielle des compositions détergentes conformes à l'invention, ces dernières contiennent en outre au moins une silicone aminée.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone aminée toute silicone comportant au moins une amine primaire, secondaire, tertiaire ou un groupement ammonium quaternaire. On peut ainsi citer :
(a) les polysiloxanes dénommés dans le dictionnaire CTFA "amodiméthicone" et répondant à la formule : 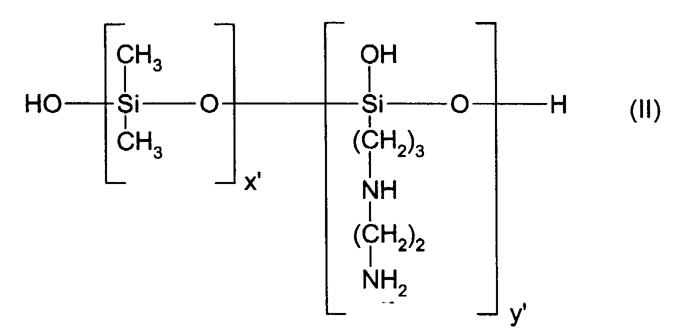 dans laquelle x' et y' sont des nombres entiers dépendant du poids moléculaire, généralement tels que ledit poids moléculaire moyen en nombre est compris entre 5 000 et 500 000 environ ;
(b) les silicones aminées répondant à la formule :

   R'ₐG₃₋ₐ-Si(OSiG₂)ₙ-(OSiG_{b}R'_{2-b})ₘ-O-SiG₃₋ₐ-R'ₐ (III)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :

      -NR"-CH₂-CH₂-N'(R")₂

      -N(R")₂

      -N^{⊕} (R")₃ A⁻

      -NH^{⊕} (R")₂ A⁻

      -NH₂^{⊕}(R") A⁻

      -N(R")-CH₂-CH₂-N^{⊕}R"H₂ A⁻,

      dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
      Un produit correspondant à cette définition est la silicone dénommée "triméthylsilylamodiméthicone", répondant à la formule : 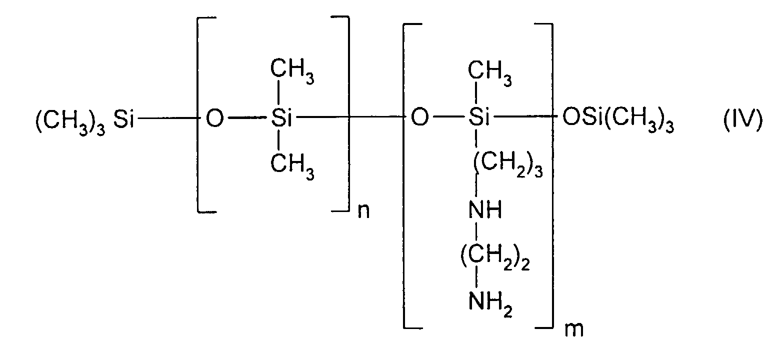 dans laquelle n et m ont les significations données ci-dessus (cf formule III).
      De tels polymères sont décrits par exemple dans la demande de brevet EP-A-95238.
(c) les silicones aminées répondant à la formule : 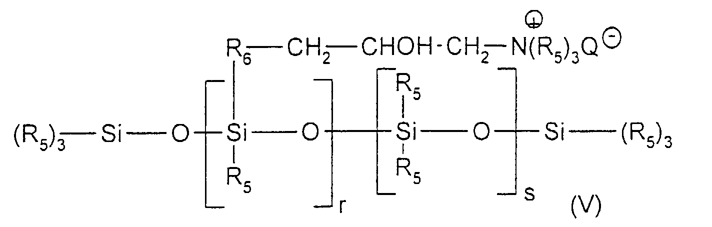 dans laquelle :
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate ...);
   r représente une valeur statistique moyenne de 2 à 20 et en particulier de 2 à 8 ; s représente une valeur statistique moyenne de 20 à 200 et en particulier de 20 à 50.

   De tels silicones aminées sont décrites plus particulièrement dans le brevet US 4 185 087.
   Une silicone entrant dans cette classe est la silicone commercialisée par la société Union Carbide sous la dénomination "Ucar® Silicone ALE 56.
d) les silicones ammonium quaternaire de formule : 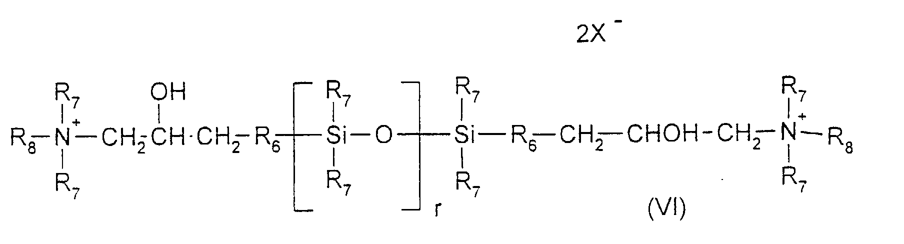 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

   Ces silicones sont par exemple décrites dans la demande EP-A-0530974.
   Des silicones entrant dans cette classe sont les silicones commercialisée par la société GOLDSCHMIDT sous les dénominations ABIL® QUAT 3270, ABIL® QUAT 3272, ABIL® QUAT 3474.
e) les silicones aminées de formule (VII) : 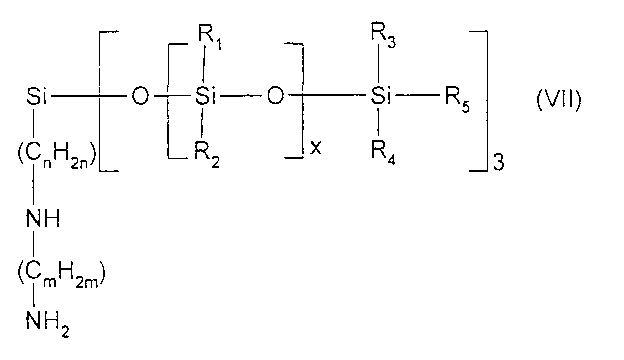 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Selon l'invention, les silicones aminées peuvent se présenter sous formes d'huile, de solutions aqueuses, alcooliques ou hydroalcooliques, sous forme de dispersion ou d'émulsion.
Une forme de réalisation particulièrement intéressante est leur utilisation sous forme d'émulsions en particulier sous forme de microémulsions ou de nanoémulsions.

On peut utiliser par exemple le produit commercialisé sous la dénomination "Emulsion Cationique DC 929" par la Société Dow Corning qui comprend, outre l'amodiméthicone, un agent de surface cationique dérivés des acides gras du suif dénomméTallowtrimonium(CTFA), en association avec un agent de surface non ionique connu sous la dénomination "Nonoxynol 10".

On peut également utiliser par exemple le produit commercialisé sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40, un autre agent de surface non ionique de formule : C₁₂H₂₅-(OCH₂-CH₂)ₙ-OH où n = 6 encore dénommé isolaureth-6, et du glycol.

### (i) Polymère(s) cationique(s) :

Au sens de la présente invention, l'expression "polymère cationique" désigne tout polymère contenant des groupements cationiques et/ou des groupements ionisables en groupements cationiques.

Les compositions conformes à l'invention comprennent en outre nécessairement un polymère cationique choisis parmi les homopolymères comportant comme constituant principal de la chaîne des motifs répondant aux formules (I) ou (I') : 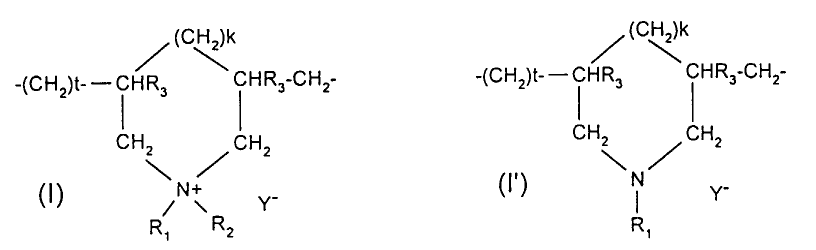 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759, dans son certificat d'addition 2.190.406 ou dans les brevets US3,996,146 et US3,288,770.

De préférence , R₁ et R₂ désignent indépendamment l'un de l'autre, méthyle ou éthyle et R₃ un atome d'hydrogène.

Les polymères cationiques utilisés ont généralement un poids moléculaire moyen en poids compris entre 5000 et 5.10⁶ environ, et de préférence comprise entre 10⁴ et 5.10⁵ environ.

Parmi les polymères définis ci-dessus, on peut citer plus particulièrement les homopolymères de chlorure de diméthyldiallylammonium tel que celui commercialisé sous la dénomination "Merquat® 100" par la société Calgon.

Les compositions conformes à l'invention renferment les silicones aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Le véhicule, ou support, des compositions détergentes selon l'invention est de préférence de l'eau ou une solution hydroalcoolique d'un alcool inférieur en C₁-C₆ tel que éthanol, isopropanol ou butanol ou un mélange d'eau et d'alkylèneglycol comme le propylèneglycol et les éthers de glycol.

Les compositions détergentes selon l'invention présentent un pH final généralement compris entre 3 et 10. De préférence, ce pH est compris entre 5,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit vendu sous la dénomination "AMINOL® A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses telles que le distéaryléther ou le 1-hexadécyloxy octadodécanol.

Les compositions conformes à l'invention peuvent éventuellement contenir en outre d'autres agents ayant pour effet d'améliorer les propriétés cosmétiques de cheveux ou de la peau. On peut citer à ce sujet les agents tensioactifs cationiques, des polymères anioniques ou non ioniques ou cationiques ou amphotères, les protéines, les hydrolysats de protéines, les céramides, les pseudocéramides, les hydroxyacides, les vitamines, le panthénol, les huiles végétales, animales, minérales ou synthétiques, les filtres solaires hydro ou liposolubles.

Ces compositions peuvent également contenir différents adjuvants utilisés couramment en cosmétique tels que des parfums, des conservateurs, des agents séquestrants, des stabilisateurs de mousse et des agents acidifiants ou alcalinisants bien connus en cosmétique.

Bien entendu, l'homme du métier veillera à choisir ce ou ces éventuels composés complémentaires et/ou leurs quantités de manière telle que les propriétés avantageuses attachées intrinsèquement à l'association (base lavante + polymère cationique spécifique + silicone aminée) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin des matières kératiniques telles que la peau ou les cheveux.

Les compositions selon l'invention sont utilisées de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage et le conditionnement des cheveux et de la peau, auquel cas ils sont appliqués sur la peau et les cheveux humides et sont rincés après application.

Les compositions conformes à l'invention sont également utilisables comme démaquillants des matières kératiniques telles que la peau, les cils, les sourcils.

L'invention a également pour objet un procédé de lavage et de conditionnement des matières kératiniques telles que les cheveux consistant à appliquer sur lesdites matières mouillées une quantité efficace d'une composition telle que définie ci-dessus, puis à effectuer un rinçage à l'eau après un éventuel temps de pause.

Des exemples concrets, mais nullement limitatifs, illustrant l'invention vont maintenant être donnés.

### EXEMPLE 1

On a réalisé deux compositions de shampooing, l'une conforme à l'invention (composition A) et l'autre comparative (composition B) :

| | A Invention | B Comparatif |
|---|---|---|
| - Lauryléthersulfate de sodium (C₁₂/C₁₄ à 70/30) à 2,2 moles d'oxyde d'éthylène en solution aqueuse à 70% de MA (MA = matière active) | 15,5 gMA | 15,5 gMA |
| - Cocobétaïne à 32% MA(*) | 3,2 gMA | ― |
| - Cocoamphodiacétate (MIRANOL® C2M CONC de RP) | ― | 3,2 gMA |
| - Polymère cationique (**) | 0,6 g | 0,6 g |
| - Silicone aminée (***) | 2,45 gMA | 2,45 |
| - Cétostéarylsulfate de sodium | 0,75 g | 0,75 g |
| - Mélange de 1-hexadécyloxy octadodécanol et d'alcool cétylique | 2,5 g | 2,5 g |
| - Alcool décylique (C₁₀/C₁₂/C₁₄) oxyéthyléné | 0,5 g | 0,5 g |
| - Acide citrique qs pH | 5 | 5 |
| - Parfum, Conservateurs | qs | qs |
| - Eau déminéralisée qs | 100 g | 100 g |

| | | |
|---|---|---|
| (*) DEHYTON® AB 30 de HENKEL | | |
| (**) : Homopolymère de chlorure de diméthyl diallyl ammonium commercialisée sous la dénomination MERQUAT® 100 (PM 400.000) par la société CALGON | | |
| (***) : Amodiméthicone commercialisée en émulsion cationique à 35% de matière active sous la dénomination FLUID DC 939 par la société DOW CORNING | | |

On effectue un shampooing en appliquant environ 12 g de la composition A sur des cheveux préalablement mouillés. On fait mousser le shampooing puis on rince abondamment à l'eau.
On procède selon le même mode opératoire que ci-dessus avec la composition comparative B.

Un panel d'experts évalue le démêlage des cheveux mouillés, le démêlage des cheveux humides séchés, la douceur et le lissage des cheveux séchés.

Tous les experts indiquent une amélioration nette de ces propriétés pour les cheveux traités avec la composition A selon l'invention.

### EXEMPLE 2

On a préparé un shampooing de composition suivante :
- Lauryléthersulfate de sodium (C₁₂/C₁₄ 70/30 en poids) à 2,2 moles d'oxyde d'éthylène en solution aqueuse à 28% de MA 14 gMA
- Cocobétaïne à 32% MA(*) 3,2 gMA
- Polymère cationique (**) 0,1 g
- Silicone aminée (***) 1,05 gMA
- Mélange de 1-hexadécyloxy 2,5 g octadodécanol et d'alcool cétylique
- Monoisopropanolamide d'acides de coprah 0,6 g
- Amino-2 méthyl-2 propanol-1 0,125 g
- Acide citrique qs pH 5,2
- Eau déminéralisée qs 100 g

(*)DEHYTON® AB 30 de HENKEL
(**) : Homopolymère de chlorure de diméthyl diallyl ammonium commercialisé sous la dénomination MERQUAT® 100 (PM 400.000) par la société CALGON
(***) : Amodiméthicone commercialisée en émulsion cationique à 35% de matière active sous la dénomination FLUID DC 939 par la société DOW CORNING

## Revendications

1. Composition détergente, caractérisée par le fait qu'elle comprend, dans un milieu cosmétiquement acceptable, (A) une base lavante comprenant au moins un tensioactif anionique choisi parmi les sels des alkyléther sulfates, alkylamidoéther sulfates, alkylaryléther sulfates; alkyléther sulfosuccinates, le radical alkyle ou acyle de tous ces différents composés comportant de préférence de 8 à 24 atomes de carbone, et le radical aryle désignant de préférence un groupement phényle ou benzyle, et au moins un tensioactif amphotère de type alkylbétaïne en C₈-C₂₀ et (B) un système conditionneur comprenant au moins une silicone aminée et au moins un polymère cationique choisi parmi les homopolymères comportant comme constituant principal de la chaîne des motifs répondant aux formules (I) et/ou (I') : 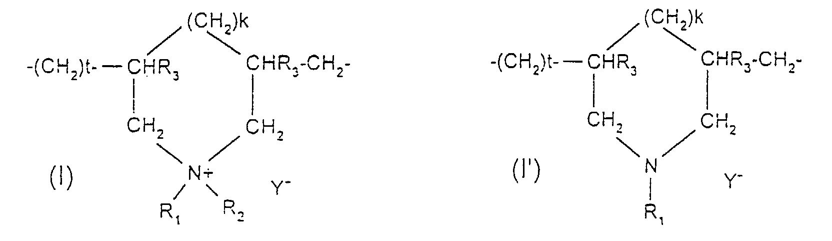 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

2. Composition selon la revendication précédente, caractérisée par le fait que R₁ et R₂, indépendamment l'un de l'autre, désignent méthyle ou éthyle et R₃ désigne un atome d'hydrogène.

3. Composition selon l'une quelconque des revendications précédentes, caractérisée par le fait que ledit polymère cationique est choisi parmi les homopolymères du chlorure de diméthyldiallylammonium.

4. Composition selon l'une quelconque des revendications précédentes, caractérisée en ce que le tensioactif anionique est choisi parmi les sels d'alkyléthersulfates en C₈-C₁₄ et plus particulièrement ceux en C₁₂-C₁₄.

5. Composition selon l'une quelconque des revendications précédentes, caractérisée en ce que le tensioactif amphotère est de type alkylbétaïne en C₁₀-C₁₄.

6. Composition selon l'une quelconque des revendications précédentes, caractérisée par le fait que la silicone aminée est choisie parmi :
(a) les polysiloxanes dénommés dans le dictionnaire CTFA "amodiméthicone" et répondant à la formule : 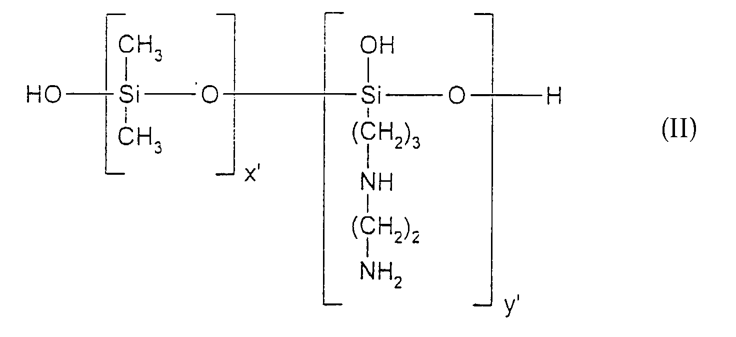 dans laquelle x' et y' sont des nombres entiers dépendant du poids moléculaire, généralement tels que ledit poids moléculaire moyen en nombre est compris entre 5 000 et 500 000 ;
(b) les silicones aminées répondant à la formule :
R'ₐG₃₋ₐ-Si(OSiG₂)ₙ-(OSiG_{b}R'_{2-b})ₘ-O-SiG₃₋ₐ-R'ₐ (III)
dans laquelle :
G est un atome d'hydrogène, ou un groupement phényle, OH, ou alkyle en C₁-C₈, par exemple méthyle,
a désigne le nombre 0 ou un nombre entier de 1 à 3, en particulier 0, b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quaternisé choisi parmi les groupements :
-NR"-CH₂-CH₂-N'(R")₂
-N(R")₂
-N^{⊕}(R")₃ A⁻
-NH^{⊕}(R")₂ A⁻
-NH₂^{⊕}(R")₃ A⁻
-N(R")-CH₂-CH₂-N^{⊕} R" H₂ A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
(c) les silicones aminées répondant à la formule : 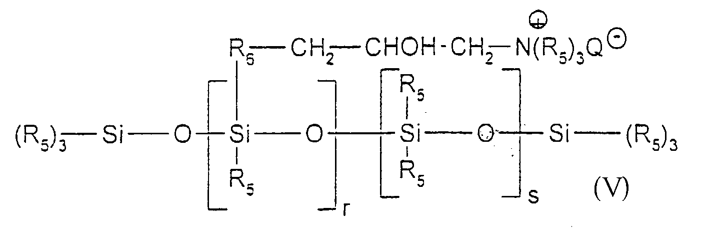 dans laquelle :
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
Q⁻ est un anion tel qu'un ion halogénure, notamment chlorure ou un sel d'acide organique (acétate ...);
r représente une valeur statistique moyenne de 2 à 20 et en particulier de 2 à 8 ; s représente une valeur statistique moyenne de 20 à 200 et en particulier de 20 à 50.
d) les silicones ammonium quaternaire de formule : 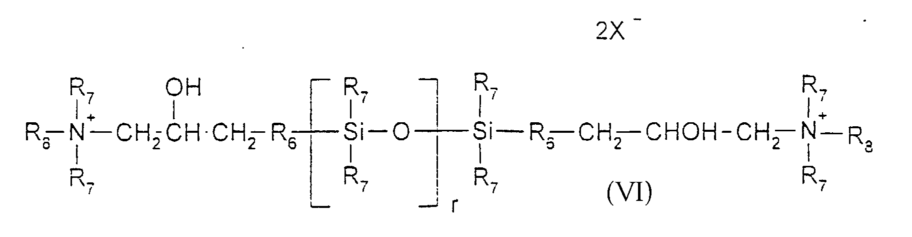 dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;
e) les silicones aminées de formule (VI) : 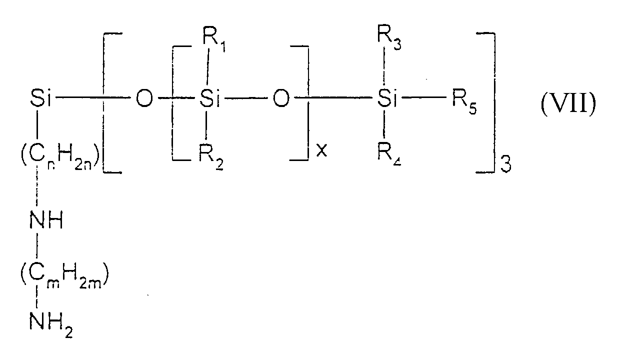 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

7. Composition selon l'une quelconque des revendications précédentes, caractérisée par le fait que le ou lesdits tensioactifs anioniques sont présents à raison de 1 à 50 % en poids, de préférence de 5 à 20 % en poids, par rapport au poids total de la composition.

8. Composition selon l'une quelconque des revendications précédentes, caractérisée par le fait que le ou lesdits tensioactifs amphotères sont présents à raison de 1 à 50 % en poids, de préférence de 1 à 20 % en poids, par rapport au poids total de la composition.

9. Composition selon l'une quelconque des revendications précédentes, caractérisée par le fait que ledit polymère cationique est présent à une teneur pondérale comprise entre 0,001 % et 10 % par rapport au poids total de la composition, de préférence entre 0,005 % et 5 % en poids et en particulier entre 0,01 % et 3 %.

10. Composition selon l'une quelconque des revendications précédentes, caractérisée par le fait que ladite silicone aminée est présente à une teneur pondérale comprise-entre 0,05 et 10% par rapport au poids total de la composition, de préférence entre 0,1 et 5 % en poids.

11. Utilisation d'une composition telle définie à l'une quelconque des revendications précédentes pour le nettoyage et/ou le conditionnement et/ou le démaquillage des matières kératiniques.

12. Procédé de lavage et de conditionnement des matières kératiniques telles que les cheveux consistant à appliquer sur lesdites matières mouillées une quantité efficace d'une composition telle que définie à l'une quelconque des revendications 1 à 10, puis à effectuer un rinçage à l'eau après un éventuel temps de pause.

## Patentansprüche

1. Reinigende Zusammensetzung, dadurch gekennzeichnet, dass sie in einem kosmetisch akzeptablen Medium (A) einen Waschrohstoff, der mindestens einen anionischen grenzflächenaktiven Stoff, der unter den Salzen von Alkylethersulfaten, Alkylamidethersulfaten, Alkylarylethersulfaten und Alkylethersulfosuccinaten ausgewählt ist, wobei die Alkylgruppe oder Acylgruppe dieser Verbindungen vorzugsweise 8 bis 24 Kohlenstoffatome aufweist und die Arylgruppe vorzugsweise Phenyl oder Benzyl bedeutet, und mindestens einen amphoteren grenzflächenaktiven Stoff vom Typ C₈₋₂₀-Alkylbetain enthält, und (B) ein Konditioniersystem aufweist, das mindestens ein aminiertes Silicon und mindestens ein kationisches Polymer enthält, das unter den Homopolymeren ausgewählt ist, die als Hauptbestandteil der Kette Einheiten der Formeln (I) und/oder (I') enthalten: 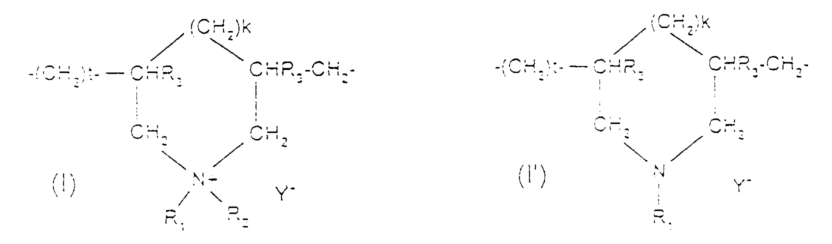 wobei in den Formeln:
- k und t Null oder 1 bedeuten,
- die Summe k + t 1 ist,
- R₃ ein Wasserstoffatom oder eine Methylgruppe bedeutet,
- R₁ und R₂ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine niedere Amidoalkylgruppe (1 bis 5 Kohlenstoffatome) bedeuten oder R₁ und R₂ zusammen mit dem Stickstoffatom, an das sie gebunden sind, heterocyclische Gruppen, wie Piperidinyl oder Morpholinyl, bedeuten können, und
- Y⁻ ein Anion bedeutet, wie beispielsweise Bromid, Chlorid, Acetat, Borat, Citrat, Tartrat, Hydrogensulfat, Hydrogensulfit, Sulfat und Phosphat.

2. Zusammensetzung nach dem vorhergehenden Anspruch, dadurch gekennzeichnet, dass R₁ und R₂ unabhängig voneinander Methyl oder Ethyl bedeuten und R₃ ein Wasserstoffatom bedeutet.

3. Zusammensetzung nach einem der vorhergehenden Ansprüche, dadurch gekennzeichnet, dass das kationische Polymer unter den Dimethyldiallylammoniumchlorid-Homopolymeren ausgewählt ist.

4. Zusammensetzung nach einem der vorhergehenden Ansprüche, dadurch gekennzeichnet, dass der anionische grenzflächenaktive Stoff unter den Salzen von Alkylethersulfaten mit 8 bis 14 Kohlenstoffatomen und insbesondere den Salzen von Alkylethersulfaten mit 12 bis 14 Kohlenstoffatomen ausgewählt ist.

5. Zusammensetzung nach einem der vorhergehenden Ansprüche, dadurch gekennzeichnet, dass der amphotere grenzflächenaktive Stoff vom Typ eines C₁₀₋₁₄-Alkylbetains ist.

6. Zusammensetzung nach einem der vorhergehenden Ansprüche, dadurch gekennzeichnet, dass das aminierte Silicon ausgewählt ist unter:
(a) Polysiloxanen, die nach CTFA-Nomenklatur als "Amodimeticon" bezeichnet werden und der folgenden Formel entsprechen: 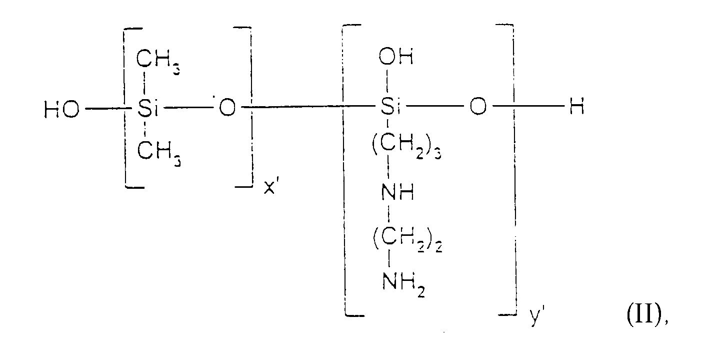 worin x' und y' ganze Zahlen sind, die vom Molekulargewicht abhängen und im allgemeinen so sind, dass das Zahlenmittel des Molekulargewichts im Bereich von 5 000 bis 500 000 liegt.
(b) Aminierten Siliconen der Formel:
R'ₐG₃₋ₐ-Si(OSiG₂)ₙ - (OSiG_{b}R'_{2-b})ₘ - O - SiG₃₋ₐ - R'ₐ (III),
worin bedeuten:
- G ein Wasserstoffatom, eine Phenylgruppe, OH oder einer C₁₋₈-Alkylgruppe, beispielsweise Methyl,
- a Null oder eine ganze Zahl von 1 bis 3 und insbesondere Null, - b Null oder 1 und insbesondere 1,
- m und n Zahlen, die so sind, dass die Summe (n + m) insbesondere im Bereich von 1 bis 2 000 und insbesondere von 50 bis 150 liegen kann, wobei n eine Zahl von 0 bis 1 999 und insbesondere von 49 bis 149 und m eine Zahl von 1 bis 2 000 und insbesondere von 1 bis 10 bedeuten kann, und
- R' eine einwertige Gruppe der Formel -C_{q}H_{2q}L, worin q eine Zahl von 2 bis 8 ist und L eine gegebenenfalls quaternisierte Aminogruppe bedeutet, die ausgewählt ist unter den Gruppen:
-NR"-CH₂-CH₂-N'(R")₂
-N(R")₂
-N^{⊕}(R")₃A⁻
-NH^{⊕}(R")₂A⁻
-NH₂^{⊕}(R")A⁻
-N(R")-CH₂-CH₂-N^{⊕}R"H₂A⁻
worin R" Wasserstoff, Phenyl, Benzyl oder eine einwertige gesättigte Kohlenwasserstoffgruppe, beispielsweise eine Alkylgruppe mit 1 bis 20 Kohlenstoffatomen, bedeuten kann und A- ein Halogenidion, wie beispielsweise Fluorid, Chlorid, Bromid oder Iodid, bedeutet.
(c) Aminierten Siliconen der Formel: 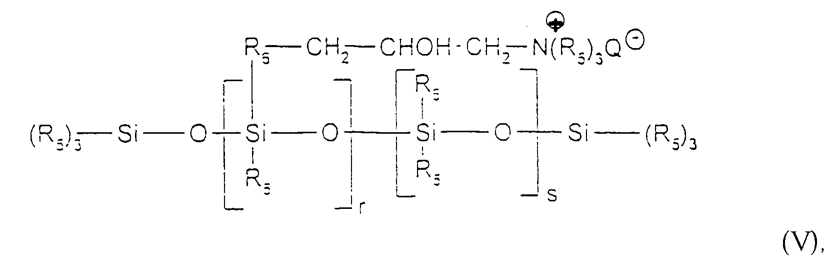 worin bedeuten:
- R₅ eine einwertige Kohlenwasserstoffgruppe mit 1 bis 18 Kohlenstoffatomen und insbesondere eine C₁₋₁₈-Alkylgruppe oder eine C₂₋₁₈-Alkenylgruppe, beispielsweise Methyl,
- R₆ eine zweiwertige Kohlenwasserstoffgruppe, insbesondere eine Alkylengruppe mit 1 bis 18 Kohlenstoffatomen oder eine zweiwertige Oxyalkylengruppe mit 1 bis 18 Kohlenstoffatomen, beispielsweise mit 1 bis 8 Kohlenstoffatomen, die durch eine SiC-Bindung an das Si gebunden ist,
- Q⁻ ein Anion, wie ein Halogenidion, insbesondere Chlorid, oder ein Salz einer organischen Säure (Acetat ...),
- r einen statistischen Mittelwert von 2 bis 20 und insbesondere von 2 bis 8 und
- s einen statistischen Mittelwert von 20 bis 200 und insbesondere von 20 bis 50.
(d) Quartären Ammoniumsiliconen der Formel: 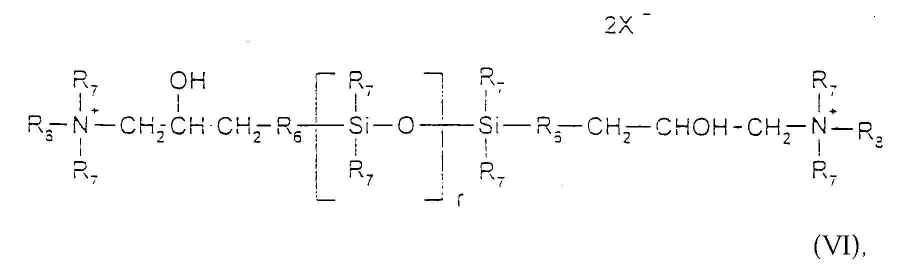 worin bedeuten:
- die Gruppen R₇, die identisch oder voneinander verschieden sind, eine einwertige Kohlenwasserstoffgruppe mit 1 bis 18 Kohlenstoffatomen und insbesondere eine C₁₋₁₈-Alkylgruppe, eine C₂₋₁₈-Alkenylgruppe oder einen Ring, der 5 oder 6 Kohlenstoffatome aufweist, beispielsweise Methyl,
- R₆ eine zweiwertige Kohlenwasserstoffgruppe und insbesondere eine Alkylengruppe mit 1 bis 18 Kohlenstoffatomen oder eine zweiwertige Oxyalkylengruppe mit 1 bis 18 Kohlenstoffatomen, beispielsweise mit 1 bis 8 Kohlenstoffatomen, die durch eine SiC-Bindung an das Si gebunden ist,
- die Gruppen R₈, die identisch oder voneinander verschieden sind, ein Wasserstoffatom, eine einwertige Kohlenwasserstoffgruppe mit 1 bis 18 Kohlenstoffatomen und insbesondere eine C₁₋₁₈-Alkylgruppe, eine C₂₋₁₈-Alkenylgruppe oder eine Gruppe -R₆-NHCOR₇,
- X⁻ ein Anion, wie ein Halogenidion, insbesondere Chlorid, oder ein Salz einer organischen Säure (Acetat ...), und
- r einen statistischen Mittelwert von 2 bis 200 und insbesondere von 5 bis 100.
(e) Aminierten Siliconen der Formel (VII): 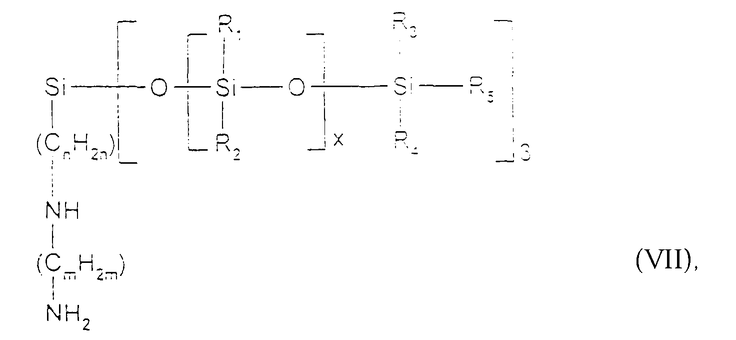 worin bedeuten:
- R₁, R₂, R₃ und R₄, die identisch oder voneinander verschieden sind, eine C₁₋₄-Alkylgruppe oder eine Phenylgruppe,
- R₅ eine C₁₋₄-Alkylgruppe oder eine Hydroxygruppe,
- n eine ganze Zahl von 1 bis 5, und
- m eine ganze Zahl von 1 bis 5,
und worin x so ausgewählt ist, dass die Aminzahl im Bereich von 0,01 bis 1 meq/g liegt.

7. Zusammensetzung nach einem der vorhergehenden Ansprüche, dadurch gekennzeichnet, dass der anionische grenzflächenaktive Stoff oder die anionischen grenzflächenaktiven Stoffe in einem Mengenanteil von 1 bis 50 Gew.-% und vorzugsweise von 5 bis 20 Gew.-%, bezogen auf das Gesamtgewicht der Zusammensetzung, vorliegen.

8. Zusammensetzung nach einem der vorhergehenden Ansprüche, dadurch gekennzeichnet, dass der amphotere grenzflächenaktive Stoff oder die amphoteren grenzflächenaktiven Stoffe in einem Mengenanteil von 1 bis 50 Gew.-% und vorzugsweise von 1 bis 20 Gew.-%, bezogen auf das Gesamtgewicht der Zusammensetzung, vorliegen.

9. Zusammensetzung nach einem der vorhergehenden Ansprüche, dadurch gekennzeichnet, dass das kationische Polymer in einem Gewichtsanteil im Bereich von 0,001 bis 10 %, vorzugsweise von 0,005 bis 5 Gew.-% und insbesondere von 0,01 bis 3 %, bezogen auf das Gesamtgewicht der Zusammensetzung, vorliegt.

10. Zusammensetzung nach einem der vorhergehenden Ansprüche, dadurch gekennzeichnet, dass das aminierte Silicon in einem Gewichtsanteil im Bereich von 0,05 bis 10 % und vorzugsweise von 0,1 bis 5 Gew.-%, bezogen auf das Gesamtgewicht der Zusammensetzung, vorliegt.

11. Verwendung einer Zusammensetzung nach einem der vorhergehenden Ansprüche zur Reinigung und/oder zum Konditionieren und/oder zum Abschminken von Keratinsubstanzen.

12. Verfahren zum Waschen und Konditionieren von Keratinsubstanzen, wie dem Haar, das darin besteht, auf die angefeuchteten Substanzen eine wirksame Menge einer Zusammensetzung nach einem der Ansprüche 1 bis 10 aufzutragen und anschließend, gegebenenfalls nach einer Einwirkzeit, mit Wasser zu spülen.

## Claims

1. Detergent composition, characterized in that it comprises, in a cosmetically acceptable medium, (A) a washing base comprising at least one anionic surfactant chosen from salts of alkyl ether sulphates, alkylamido ether sulphates, alkylaryl ether sulphates; alkyl ether sulphosuccinates, the alkyl or acyl radical of all these various compounds preferably containing from 8 to 24 carbon atoms, and the aryl radical preferably denoting a phenyl or benzyl group, and at least one amphoteric surfactant of C₈-C₂₀ alkylbetaine type and (B) a conditioning system comprising at least one aminosilicone and at least one cationic polymer chosen from homopolymers containing, as main constituent of the chain, units corresponding to formulae (I) and/or (I'): 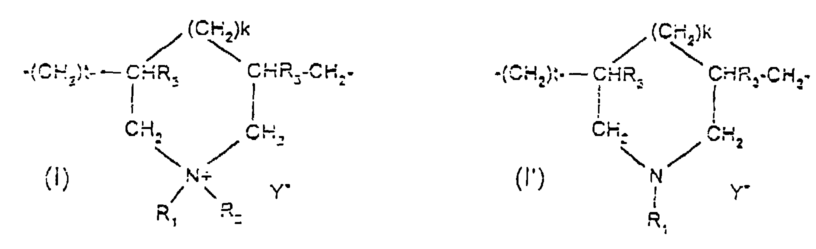 in which formulae k and t are equal to 0 or 1, the sum k + t being equal to 1; R₃ denotes a hydrogen atom or a methyl radical; R₁ and R₂, independently of each other, denote an alkyl group having from 1 to 22 carbon atoms, a hydroxyalkyl group in which the alkyl group preferably has 1 to 5 carbon atoms, a lower (1 to 5 carbon atoms) amido alkyl group, or R₁ and R₂ can denote, together with the nitrogen atom to which they are attached, heterocyclic groups such as piperidyl or morpholinyl; Y⁻ is an anion such as bromide, chloride, acetate, borate, citrate, tartrate, bisulphate, bisulphite, sulphate or phosphate.

2. Composition according to the preceding claim, characterized in that R₁ and R₂, independently of each other, denote methyl or ethyl and R₃ denotes a hydrogen atom.

3. Composition according to either of the preceding claims, characterized in that the said cationic polymer is chosen from dimethyldiallylammonium chloride homopolymers.

4. Composition according to any one of the preceding claims, characterized in that the anionic surfactant is chosen from C₈-C₁₄, and more particularly C₁₂-C₁₄, alkyl ether sulphate salts.

5. Composition according to any one of the preceding claims, characterized in that the amphoteric surfactant is of C₁₀-C₁₄ alkylbetaine type.

6. Composition according to any one of the preceding claims, characterized in that the aminosilicone is chosen from:
(a) the polysiloxanes referred to in the CTFA dictionary as "amodimethicone" and corresponding to the formula: 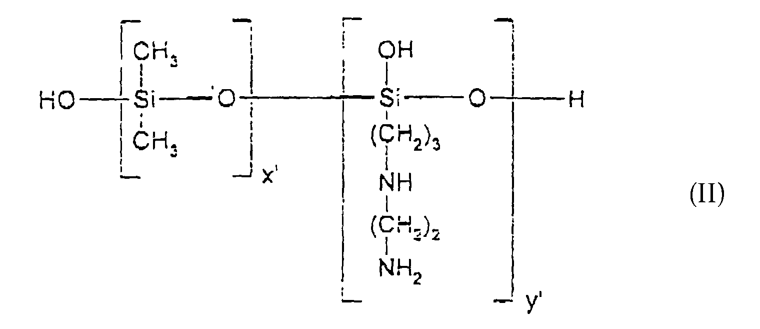 in which x' and y' are integers dependent on the molecular weight, generally such that the said number-average molecular weight is between 5 000 and 500 000;
(b) aminosilicones corresponding to the formula:
R'ₐG₃₋ₐ-Si(OSiG₂)ₙ-(OSiG_{b}R'_{2-b})ₘ-O-SiG₃₋ₐ-R'ₐ (III)
in which:
G is a hydrogen atom or a phenyl, OH or C₁-C₈ alkyl group, for example methyl,
a denotes the number 0 or an integer from 1 to 3, in particular 0,
b denotes 0 or 1, and in particular 1,
m and n are numbers such that the sum (n + m) can range especially from 1 to 2 000 and in particular from 50 to 150, it being possible for n to denote a number from 0 to 1 999 and especially from 49 to 149 and it being possible for m to denote a number from 1 to 2 000 and especially from 1 to 10;
R' is a monovalent radical of formula -C_{q}H_{2q}L in which q is a number from 2 to 8 and L is an optionally quaternized amine group chosen from the groups:
-NR"-CH₂-CH₂-N' (R")₂
-N(R")₂
-N^{⊕}(R")₃A⁻
-NH^{⊕} (R")₂A⁻
-NH₂^{⊕} (R")A⁻
-N (R")-CH₂-CH₂-N^{⊕}R"H₂A⁻,
in which R" can denote hydrogen, phenyl, benzyl, or a saturated monovalent hydrocarbon radical, for example an alkyl radical having from 1 to 20 carbon atoms, and A⁻ represents a halide ion such as, for example, fluoride, chloride, bromide or iodide.
(c) aminosilicones corresponding to the formula: 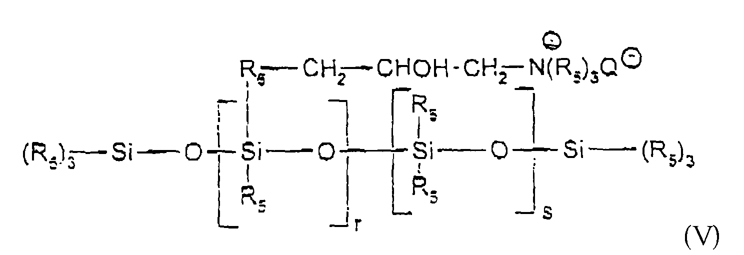 in which:
R₅ represents a monovalent hydrocarbon radical having from 1 to 18 carbon atoms, and in particular a C₁-C₁₈ alkyl or C₂-C₁₈ alkenyl radical, for example methyl;
R₆ represents a divalent hydrocarbon radical, in particular a C₁-C₁₈ alkylene radical or a divalent C₁-C₁₈, for example C₁-C₈, alkylenoxy radical connected to the Si by an SiC bond;
Q⁻ is an anion such as a halide ion, in particular chloride, or an organic acid salt (acetate);
r represents an average statistical value from 2 to 20 and in particular from 2 to 8;
s represents an average statistical value from 20 to 200 and in particular from 20 to 50.
d) quaternary ammonium silicones of formula: 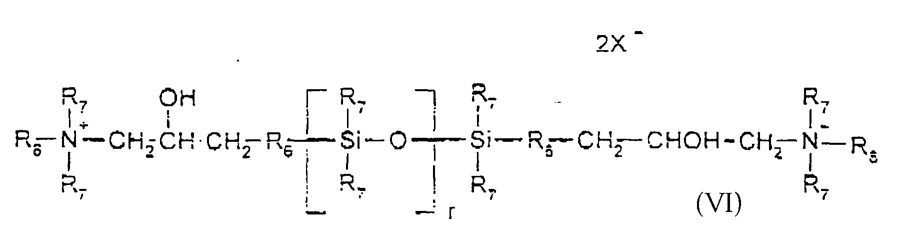 in which:
R₇, which may be identical or different, represent a monovalent hydrocarbon radical having from 1 to 18 carbon atoms, and in particular a C₁-C₁₈ alkyl radical, a C₂-C₁₈ alkenyl radical or a ring comprising 5 or 6 carbon atoms, for example methyl;
R₆ represents a divalent hydrocarbon radical, in particular a C₁-C₁₈ alkylene radical or a divalent C₁-C₁₈, for example C₁-C₈, alkylenoxy radical connected to the Si by an SiC bond;
R₈, which may be identical or different, represent a hydrogen atom, a monovalent hydrocarbon radical having from 1 to 18 carbon atoms, and in particular a C₁-C₁₈ alkyl radical, a C₂-C₁₈ alkenyl radical or a radical -R₆-NHCOR₇ ;
X⁻ is an anion such as a halide ion, in particular chloride, or an organic acid salt (acetate, etc.); r represents an average statistical value from 2 to 200 and in particular from 5 to 100.
e) aminosilicones of formula (VII) : 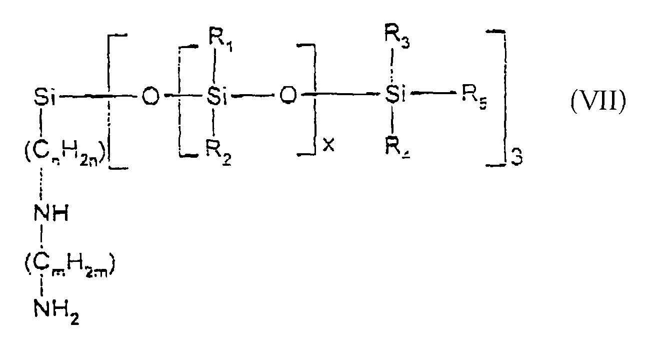 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5,
and in which x is chosen such that the amine number is between 0.01 and 1 meq/g.

7. Composition according to any one of the preceding claims, characterized in that the said anionic surfactant(s) is(are) present in a proportion of from 1% to 50% by weight, preferably from 5% to 20% by weight, relative to the total weight of the composition.

8. Composition according to any one of the preceding claims, characterized in that the said amphoteric surfactant(s) is(are) present in a proportion of from 1% to 50% by weight, preferably from 1% to 20% by weight, relative to the total weight of the composition.

9. Composition according to any one of the preceding claims, characterized in that the said cationic polymer is present in a weight content of between 0.001% and 10% relative to the total weight of the composition, preferably between 0.005% and 5% by weight and in particular between 0.01% and 3%.

10. Composition according to any one of the preceding claims, characterized in that the said aminosilicone is present in a weight content of between 0.05% and 10% relative to the total weight of the composition, preferably between 0.1% and 5% by weight.

11. Use of a composition as defined in any one of the preceding claims, for cleaning and/or conditioning and/or removing make-up from keratin materials.

12. Process for washing and conditioning keratin materials such as the hair, which consists in applying an effective amount of a composition as defined in any one of Claims 1 to 10 to the said wet substances, followed by rinsing with water after optionally leaving the composition on the keratin materials for a while.
